# EUROPEAN PATENT SPECIFICATION

(11) **EP 0 460 034 B1**
(45) Date of publication and mention of the grant of the patent: **27.04.1994**
(21) Application number: 90903795.4
(22) Date of filing: 13.02.1990
(51) Int. Cl.: C07C 401/00, C07F 7/18, A61K 31/59

(54) **NOVEL VITAMIN D ANALOGUES**
VITAMIN D ANALOGE
NOUVEAUX ANALOGUES DE VITAMINE D

(30) Priority: 23.02.1989 GB 8904153
(43) Date of publication of application: 11.12.1991
(73) Proprietor: LEO PHARMACEUTICAL PRODUCTS LTD. A/S (LOVENS KEMISKE FABRIK PRODUKTIONSAKTIESELSKAB), 2750 Ballerup (DK)
(72) Inventor: CALVERLEY, Martin, John, DK-2730 Herlev (DK); HANSEN, Kai, DK-2730 Herlev (DK); BINDERUP, Lise, DK-2630 Tastrup (DK)
(74) Representative: Kinzebach, Werner, Dr.
(86) International application number: DK9000037
(87) International publication number: WO9009992

(56) References cited:
- EP-A- 0 184 112
- CHEM. PHARM. BULL., vol. 34, no. 10, 1986; E. MURAYAMA et al, "Synthetic studies of vitamin D3 analogues. VIII. Synthesis of 22-oxavitamin D3 analogues", pp. 4410-4413
- CHEMICAL ABSTRACTS, vol. 110, no. 2, 9 January 1989, Columbus, Ohio, US; K. MIYAMOTO et al, "Antitumor pharmaceuticals containing 9,10-seco-5,7,10(19)-pregnatriene derivatives", abstract no. 13599s
- CHEMICAL ABSTRACTS; vol. 108, no. 11, 14 March 1988, Columbus, Ohio, US; J. ABE et al, "Synthetic analogs of vitamin D3 with an oxygen atom in the side chain skeleton. A trial of the development of vitamin D compounds which exhibit potent differentiation-inducing activity without inducing hypercalcemia", abstract no. 88191v

## Description

This invention relates to a hitherto unknown class of compounds which shows an immunomodulating effect as well as strong activity in inducing differentiation and inhibiting undesirable proliferation of certain cells, including cancer cells and skin cells, to pharmaceutical preparations containing these compounds, to dosage units of such preparations, and to their use in the treatment and prophylaxis of autoimmune diseases, including diabetes mellitus, hypertension, inflammatory diseases such as rheumatoid arthritis and asthma as well as diseases characterized by abnormal cell differentiation and/or cell proliferation, and/or imbalance in the immune system.

The compounds of the present invention are represented by the general formula I 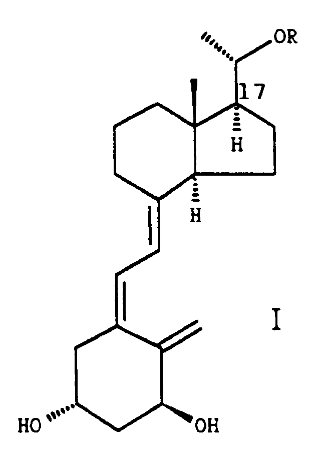
in which formula R stands for an alkyl group containing from 7 to 12 carbon atoms optionally substituted with a hydroxy group.

Preferably R is a group of formula II 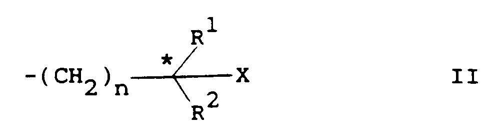
where n is an integer from 1 to 7; R¹ and R², which may be the same or different, stand for hydrogen, lower alkyl, lower cycloalkyl, or taken together with the carbon atom (starred in formula II) bearing the group X, R¹ and R² can form a C₃-C₈ carbocyclic ring; X stands for hydroxy or hydrogen.

In the context of this invention, the expression "lower alkyl" indicates a straight or branched saturated or unsaturated carbon chain containing from 1 to 5 carbon atoms, and the expression "lower cyclo-alkyl" indicates a saturated or unsaturated C₃-C₇ carbocyclic ring.

As can be seen from formula I and II, depending on the meanings of R, X, R¹ and R², the compounds of the invention can comprise several diastereoisomeric forms (e.g. R or S configuration at the starred carbon atom). The invention covers all these diastereoisomers in pure form and also mixtures of diastereoisomers. In addition, derivatives of I in which one or more of the hydroxy groups are masked as groups which can be reconverted to hydroxy groups in vivo are also within the scope of the invention ("bioreversible derivatives or prodrugs of I").

The term "bioreversible derivatives or prodrugs of I" includes, but is not limited to, derivatives of the compounds of formula I in which one or more hydroxy groups have been transformed into -O-acyl or -O-glycosyl groups, or a phosphate ester, such masked groups being hydrolyzable in vivo.

The compounds I in which R is not substituted with hydroxy are another type of prodrug. These compounds are relatively inactive in vitro, but are converted to active compounds of formula I by enzymatic hydroxylation after administration to the patient.

It has recently been shown that 1α,25-dihydroxy-vitamin D₃ (1,25(OH)₂D₃) influences the effects and/or production of interleukins, indicating the potential use of this compound in the treatment of diseases characterized by a dysfunction of the immune system, e.g. autoimmune diseases and rejection of transplants. In addition, other conditions characterized by an abnormal interleukin-1 production, e.g. inflammatory diseases such as rheumatoid arthritis may be treated with 1,25(OH)₂D₃.

It has also been shown that 1,25(OH)₂D₃ is able to stimulate the differentiation of cells and inhibit excessive cell proliferation, and it has been suggested that this compound might be useful in the treatment of diseases characterized by abnormal cell proliferation and/or cell differentiation such as cancer and psoriasis.

Also, the use of 1,25(OH)₂D₃ for the treatment of hypertension and diabetes mellitus has been suggested.

However, the therapeutic possibilities in such indications of 1,25(OH)₂D₃ are severely limited by the well known potent effect of this hormone on calcium metabolism; elevated blood concentrations will rapidly give rise to hypercalcemia. Thus, this compound and its potent synthetic analogues are not completely satisfatory for use as drugs in the treatment of e.g. psoriasis, cancer or immune diseases which may require continuous administration of the drug in relatively high doses.

The 22-oxa-analogues of 1α-hydroxyvitamin D₃ and 1α,25-dihydroxyvitamin D₃ and are described in E. Murayama et al, Chem. Pharm. Bull., 34, 4410 (1986), J. Abe et al, FEBS LETTER, 226, 58 (1987) and European Patent Application, publication number 184 112.

In vitro experiments indicate that a 22-oxa analogue may have advantageous properties. Thus 1α,25-dihydroxy-22--oxavitamin D₃ is reported to have only one 14th as much affinity as 1α,25(OH)₂D₃ for the chick intestinal cytosolic receptor, a weaker affinity than 1,25(OH)₂D₃ for the receptor in a human myeloid leukemia cell line (HL-60), but high activity as an inducer of differentiation in HL-60 cells.

The usefulness of a vitamin D analogue in the above mentioned indications is dependent not only upon a favourable ratio of binding affinity to relevant receptors compared to the intestinal receptor, but also upon the fate of the compound in the organism.

These known 22-oxa compounds are characterized by having a side chain (the substituent on C-17 (confer formula I) whose size (total number of carbon atoms) does not exceed that in 1,25-(OH)₂D₃ itself (i.e. 8 carbon atoms). We have now found to our surprise that the analogues of type I having at least 7 carbon atoms in the group R (i.e. a total of at least 9 carbon atoms in the side chain) exhibit a combination of metabolic stability, favourable selectivity with respect to the binding affinities to various receptors, and reduced calcemic effects which makes them significantly superior to the hereto known 22-oxa compounds.

The selectivity of the compounds of the invention is illustrated by the fact that while they have high affinities for the receptor in tumour cells (similar to or better than that of 1,25(OH)₂D₃) and the concentration needed to induce cell differentiation in a human monocytic tumour cell line is the same as or lower than that needed of 1,25(OH)₂D₃ to give the same effect, their binding affinity for the intestinal receptor is much lower than that of 1,25(OH)₂D₃. In vivo in rats the compounds are considerably less active than 1,25(OH)₂D₃ in inducing hypercalciuria and hypercalcemia.

For example, direct comparison (cf. Biochem. Pharmacol. 1988, 37, 889) of the compound I of Example 1 with 22-oxa-1,25(OH)₂D₃ has shown that the former is more potent an inhibitor of the proliferation of U937 cells, binds less strongly to the rachitic chick intestinal receptor, and is less potent in its calcemic effects as reflected by the concentration required to elevate serum calcium concentration in normal rats.

This renders the compounds of the invention especially suited for both local and systemic treatment and prophylaxis of human and veterinary disorders which are characterized by abnormal cell proliferation and/or cell differentiation, such as certain dermatological disorders including psoriasis and certain cancer forms, e.g. leukemia and myelofibrosis, and diseases characterized by an imbalance in the immune system, e.g autoimmune diseases, or AIDS, and to obtain desired immunosuppression as in transplantation procedures, as well as treatment of acne, diabetes mellitus and hypertension and inflammatory diseases, such as rheumatoid arthritis and asthma. As the compounds of this invention may promote the differentiation of the hair follicle cells, these compounds may be used in the treatment of alopecia.

The compounds of formula I may conveniently be prepared from the vitamin D-derivative 1 (or its 20R isomer) (Tetrahedron, 43, 4609 (1987)) by the routes outlined in Scheme 1. Oxidation of 1 for example using the van Rheenen procedure (Tetrahedron Letters, 1969, 985) gives the ketone 2, which is reduced to the 20S-alcohol 3. When a suitable chiral reducing agent (for example an organoboron reagent) is used, 3 may be prepared stereoselectively, (cf. S. Masamune in "Stereochemistry of Organic and Bioorganic Transformations", ex W. Bartmann and K.B. Sharpless, eds., VCH, Weinheim, 1987, p. 60). Alternatively, 3 is conveniently prepared by NaBH₄ reduction of 2 and separating the co-produced major amount of corresponding 20R-alcohol chromatographically. O-Alkylation of 3 to give III is achieved by treatment under basic conditions with a side chain building block of general formula Z-R³, in which Z is a leaving group such as a halogen (Cl, Br or I) or p-toluenesulphonyloxy or methanesulphonyloxy, and R³ is R (of formula I) or optionally a radical which can be converted to R at any convenient later stage (or over several stages). Thus R³ in compounds III, IV, V and VI does not necessarily have the same meaning along a particular synthetic sequence. The conversion of R³ to R may well involve several steps and possibly involve a temporary protection of the sensitive triene system of the molecule. Apart from any necessary modification within the side chain (R³), the conversion of III to I involves a photoisomerisation step and a desilylation step, analogous to the steps used in the last stages of the synthesis of other vitamin D analogues (see European patent No. 0 227 826).

The side chain building blocks, R³Z, are either known compounds (several are described in international patent application PCT/DK89/00079) or may be prepared analogously to those described in PCT/DK89/00079. The R³ is typically identical with formula II in which X is a protected OH group, e.g. tetrahydropyranyloxy or trialkylsilyloxy. (Any such THP ethers R³Z, which are not described in PCT/DK89/00079, are readily prepared from the corresponding alcohol).

The following standard abbreviations are used throughout this disclosure: Me = methyl; Et = ethyl; Prⁿ = n-propyl; Bu^{t} = tert-butyl; THP = tetrahydro-4H-pyran-2-yl; THF = tetrahydrofuran: Ts = p-toluenesulphonyl; TBA = tetra-(n-butyl)-ammonium. 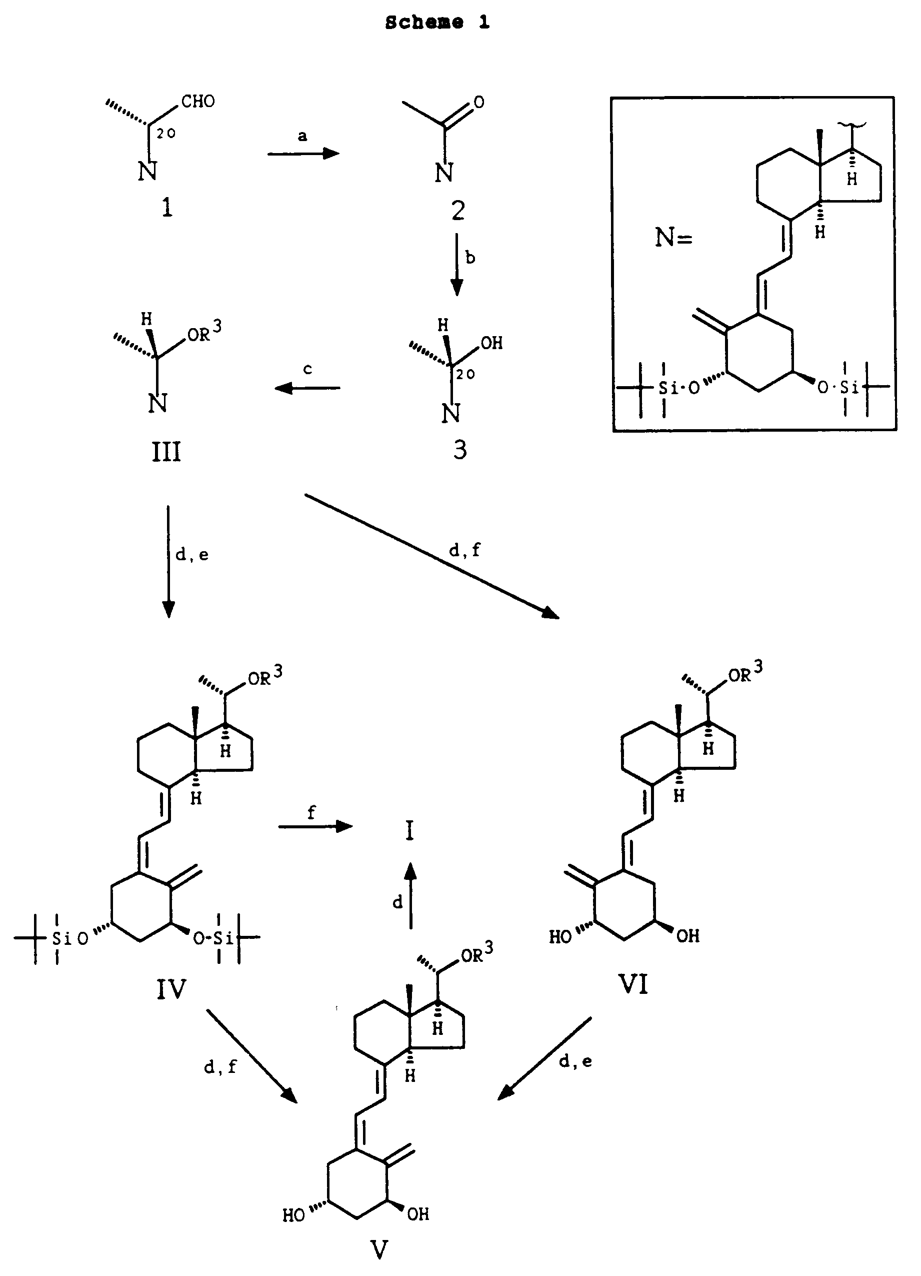
An alternative approach to the synthesis of compounds I via compounds IV of Scheme 1 is shown in Scheme 2. In this Scheme, the aldehyde 4 (Wovkulich et al, Tetrahedron 1984, 40, 2283) is degraded stereoselectively to the alcohol 8 via the intermediates shown. Alkylation of 8 to give VII is analogous to the reaction 3 → III of Scheme 1. Together with appropriate modification of R³ (the meaning of which may be changed along the reaction sequence to a particular compound I), conversion of VII to VIII, Wittig coupling with the anion derived from IX (cf. Wovkulich, op. cit.) and deprotection gives the target compound I.

The compounds 3 and 8 are very versatile intermediates not only for the preparation of compounds I of the invention, but also for other analogues of formula I in which the group R is excluded from the present invention, such as the previously known 22-oxa-1,25-(OH)₂D₃. Indeed, we have conveniently used the reactions of both Schemes 1 and 2 for the synthesis of reference samples of 22-oxa-1,25-(OH)₂D₃ for direct biological comparison with the compounds of the invention.

### Notes to Schemes 1 and 2

a) Oxidation with O₂ e.g. with Cu(AcO)₂, 2,2'-bipyridyl and 1,4-diazabicyclo[2,2,2]octane as catalyst.
b) Reduction (e.g. with NaBH₄).
c) Alkylation with the side chain building block R³-Z in the presence of base (e.g. KOH, KOBu^{t} or KH, with or without catalyst (e.g. 18-Crown-6) in solvent, e.g. THF).
d) Optional functional group modification in the side chain.
e) Isomerisation with hν - triplet sensitizer, e.g. anthracene.
f) Deprotection (e.g. with TBA⁺F⁻ or HF).
g) MeMgI or MeLi.
h) Oxidation (e.g. with pyridinium dichromate)
i) Baeyer-Villiger an oxidation (e.g. with m-chloroperbenzoic acid)
j) Hydrolysis or alcoholysis (e.g. KOH in MeOH).
k) (i) Desilylation (e.g. HF); (ii) Oxidation (e.g. pyridinium chlorochromate).
l) Reaction with the anion (e.g. lithio-derivative) derived by treating IX with an equivalent amount of base (e.g. n-BuLi).

It should be noted that although the shown intermediates may have hydroxyl groups protected as tert-butyl-dimethylsilyl ethers, the scope of the invention does not exclude the use of alternative hydroxyl protecting groups well known in the art (such as those described in T.W. Greene, "Protective groups in organic synthesis", Wiley, New York, 1981), together with alternative reactions for deprotection. 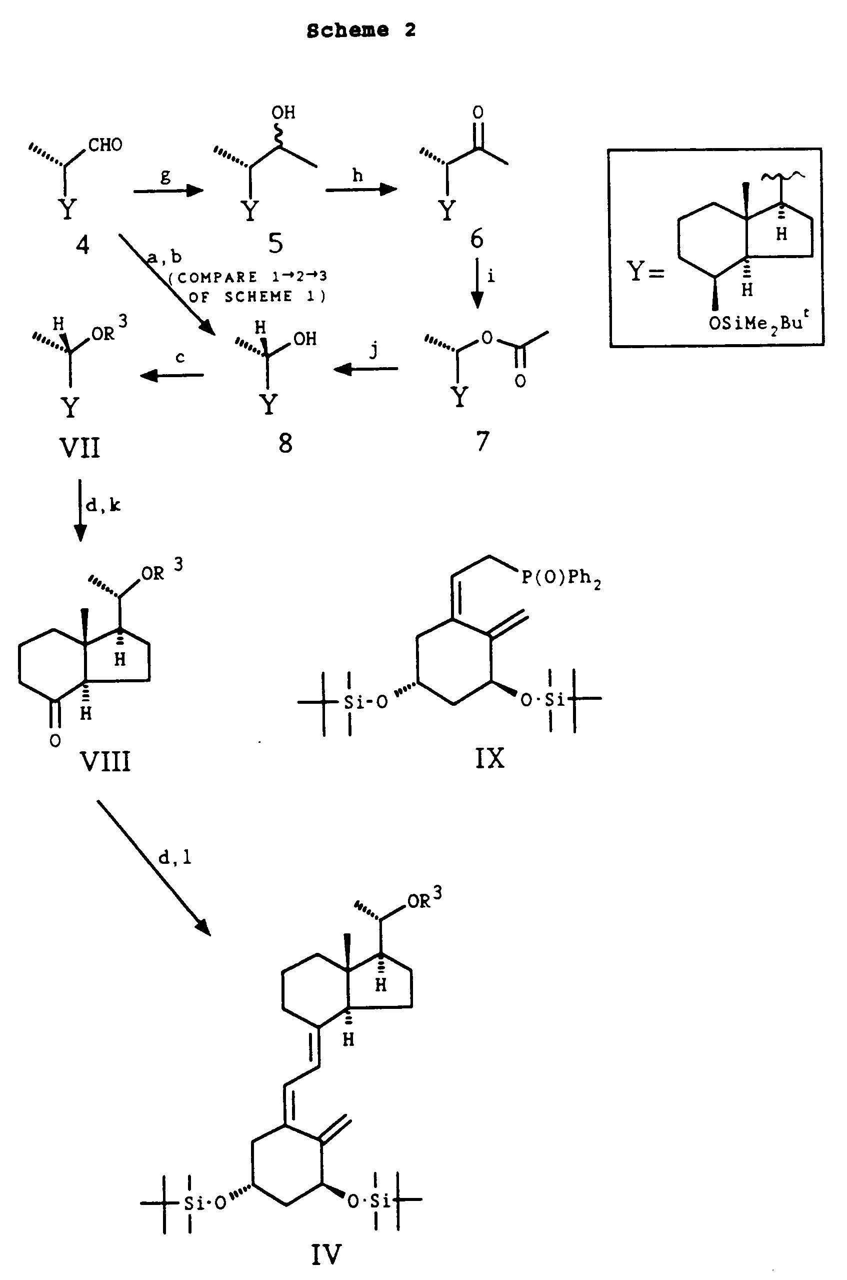
The present compounds are intended for use in pharmaceutical compositions which are useful in the treatment of human and veterinary disorders as described above.

The amount required of a compound of formula I (hereinafter referred to as the active ingredient) for therapeutic effect will, of course, vary both with the particular compound, the route of administration and the mammal under treatment. The compounds of the invention can be administered by the parenteral, intra-articular, enteral or topical routes. They are well absorbed when given enterally and this is the preferred route of administration in the treatment of systemic disorders. In the treatment of dermatological disorders like psoriasis, topical or enteral forms are preferred.

In the treatment of respiratory diseases like asthma an aerosol is preferred.

While it is possible for an active ingredient to be administered alone as the raw chemical, it is preferable to present it as a pharmaceutical formulation. Conveniently, the active ingredient comprises from 1 ppm to 0.1% by weight of the formulation.

By the term "dosage unit" is meant a unitary, i.e. a single dose which is capable of being administered to a patient, and which may be readily handled and packed, remaining as a physically and chemically stable unit dose comprising either the active material as such or a mixture of it with solid or liquid pharmaceutical diluents or carriers.

The formulations, both for veterinary and for human medical use, of the present invention comprise an active ingredient in association with a pharmaceutically acceptable carrier therefore and optionally other therapeutic ingredient(s). The carrier(s) must be "acceptable" in the sense of being compatible with the other ingredients of the formulations and not deleterious to the recipient thereof.

The formulations include e.g. those in a form suitable for oral, rectal, parenteral (including subcutaneous, intramuscular and intravenous), intra-articular and topical administration.

The formulations may conveniently be presented in dosage unit form and may be prepared by any of the methods well known in the art of pharmacy. All methods include the step of bringing the active ingredient into association with the carrier which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be administered in the form of a bolus, electuary or paste.

A tablet may be made by compressing or moulding the active ingredient optionally with one or more accessory ingredients. Compressed tablets may be prepared by compressing, in a suitable machine, the active ingredient in a free-flowing form such as a powder or granules, optionally mixed by a binder, lubricant, inert diluent, surface active or dispersing agent. Moulded tablets may be made by moulding, in a suitable machine, a mixture of the powdered active ingredient and suitable carrier moistened with an inert liquid diluent.

Formulations for rectal administration may be in the form of a suppository incorporating the active ingredient and a carrier such as cocoa butter, or in the form of an enema.

Formulations suitable for parenteral administration conveniently comprise a sterile oily or aqueous preparation of the active ingredient which is preferably isotonic with the blood of the recipient.

Formulations suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical administration include liquid or semi-liquid preparations such as liniments, lotions, applicants, oil-in-water or water-in-oil emulsions such as creams, ointments or pastes; or solutions or suspensions such as drops.

For asthma treatment inhalation of powder, self-propelling or spray formulations, dispensed with a spray can, a nebulizer or an atomizer can be used. The formulations, when dispensed, preferably have a particle size in the range of 10 to 100 µ.

Such formulations are most preferably in the form of a finely comminuted powder for pulmonary administration from a powder inhalation device or self-propelling powder-dispensing formulations. In the case of self-propelling solution and spray formulations, the effect may be achieved either by choice of a valve having the desired spray characteristics (i.e. being capable of producing a spray having the desired particle size) or by incorporating the active ingredient as a suspended powder in controlled particle size. These selfpropelling formulations may be either powder-dispensing formulations or formulations dispensing the active ingredient as droplets of a solution or suspension.

Self-propelling powder-dispensing formulations preferably comprise dispersed particles of solid active ingredients, and a liquid propellant having a boiling point below 18°C at atmospheric pressure. The liquid propellant may be any propellant known to be suitable for medicinal administration and may comprise one or more C₁-C₆-alkyl hydrocarbons or halogenated C₁-C₆-alkyl hydrocarbons or mixtures thereof; chlorinated and flourinated C₁-C₆-alkyl hydrocarbons are especially preferred. Generally, the propellant constitutes 45 to 99.9% w/w of the formulation whilst the active ingredient constitutes 1 ppm to 0.1% w/w, of the formulation.

In addition to the aforementioned ingredients, the formulations of this invention may include one or more additional ingredients such as diluents, buffers, flavouring agents, binders, surface active agents, thickeners, lubricants, preservatives, e.g. methyl hydroxybenzoate (including anti-oxidants), emulsifying agents and the like.

The compositions may further contain other therapeutically active compounds usually applied in the treatment of the above mentioned pathological conditions.

The present invention further concerns a method for treating patients suffering from one of the above pathological conditions, said method consisting of administering to a patient in need of treatment an effective amount of one or more compounds of formula I, alone or in combination with one or more other therapeutically active compounds usually applied in the treatment of said pathological conditions. The treatment with the present compounds and/or with further therapeutically active compounds may be simultaneous or with intervals.

In the treatment of systemic disorders daily doses of from 1-1000 µg, preferably from 2-250 µg, of a compound of formula I are administered. In the topical treatment of dermatological disorders, ointments, creams or lotions containing from 1-1000 µg/g, and preferably from 10-500 µg/g, of a compound of formula I are administered. The oral compositions are formulated, preferably as tablets, capsules, or drops, containing from 0.5-500 µg, preferably from 1-250 µg, of a compound of formula I, per dosage unit.

The invention will now be further described in the following non-limiting Preparations and Examples:

### Preparations and Examples

### General

The exemplified compounds I are listed in Table 1. The intermediates of Schemes 1 and 2 referred to in the Preparations are to be identified by numbers with the corresponding formulae in Table 2.

For nuclear magnetic resonance spectra (300 MHz) chemical shift values (δ) are quoted for deuteriochloroform solutions relative to internal tetramethylsilane (δ = 0) or chloroform (δ = 7.25). The value for a multiplet, either defined (doublet (d), triplet (t), quartet (q)) or not (m) at the approximate mid point is given unless a range is quoted (s = singlet, b = broad). Coupling constants (J) are given in Hertz, and are sometimes approximated to the nearest unit.

Ether is diethyl ether, and was dried over sodium. THF was dried over sodium-benzophenone. Petroleum ether refers to the pentane fraction. Reactions were run at room temperature unless otherwise noted. The work-up procedure referred to involves dilution with the specified solvent (otherwise the organic reaction solvent), extraction with water and then brine, drying over anhydrous MgSO₄, and concentration in vacuo to give a residue.

**Table 1**

| Exemplified Compounds I | | | | | |
|---|---|---|---|---|---|
| Compound Number | Example Number | Formula (II) | | | |
| | | n | R¹ | R² | X |
| 101 | 1 | 4 | Me | Me | OH |
| 102 | 2 | 3 | Et | Et | OH |
| 103 | 3 | 5 | Me | Me | OH |

**Table 2**

| Compound Number | Preparation Number | Formula | |
|---|---|---|---|
| | | Type (See Schemes) | R³ |
| 9 | 5 | III | -(CH₂)₄-C(O-THP)Me₂ |
| 10 | 6 | III | -(CH₂)₃-C(OSiMe₃)Et₂ |
| 11 | 7 | III | -(CH₂)₅-C(OSiMe₃)Me₂ |
| 12 | 9 | IV | -(CH₂)₄-C(O-THP)Me₂ |
| 13 | 10 | IV | -(CH₂)₃-C(OSiMe₃)Et₂ |
| 14 | 11,12 | IV | -(CH₂)₅-C(OSiMe₃)Me₂ |
| 15 | 8 | VII | -(CH₂)₅-C(OSiMe₃)Me₂ |

### Preparation 1: Compound 2

To a solution of 1(S),3(R)-bis-(tert-butyldimethylsilyloxy)-20(S)-formyl-9,10-secopregna-5(E),(7E),10(19)-triene (Compound 1) (3.44 g, 6 mmol) in N,N-dimethylformamide (150 ml), 1,4-diazabicyclo[2.2.2]octane (600 mg, 5.3 mmol), cupric acetate, monohydrate (90 mg, 0.45 mmol) and 2,2'-bipyridyl (72 mg, 0.45 mmol) were added. Air was bubbled through the well stirred solution for 6 days at 40°C.

The reaction mixture was diluted with ethyl acetate (500 ml), extracted with water (2 x 100 ml) and saturated aqueous sodium chloride (3 x 50 ml) and dried over MgSO₄. Ethyl acetate was evaporated off, and the solid residue was purified by chromatography (silica gel, 10% ether in petroleum ether as eluant) to give the title compound.

NMR: δ = 0.037 (s, 3H), 0.043 (s, 3H), 0.056 (s, 6H), 0.49 (s, 3H), 0.84 (s, 9H), 0.89 (s, 9H), 1.5-2.30 (m, 13H), 2.13 (s, 3H), 2.55 (dd, 1H), 2.70 (t, 1H), 2.89 (bd, 1H), 4.21 (m, 1H), 4.52 (m, 1H), 4.94 (m, 1H), 4.98 (m, 1H), 5.83 (d, 1H), 6.43 (d, 1H) ppm.

### Preparation 2: Compound 3 and its 20R-isomer

Compound 2 (Prep. 1) (3.10 g, 5.5 mmol) was dissolved in tetrahydrofuran (140 ml) and sodium borohydride (0.35 g, 3.3 mmol) was added. Methanol was then added dropwise over 15 minutes. The reaction blend was stirred for 20 minutes, then diluted with ethyl acetate (560 ml). The solution was extracted with water (5 x 150 ml) and saturated aqueous sodium chloride (150 ml), dried over MgSO₄ and evaporated to give a colourless oil. The oily residue was purified by chromatography (silica gel, 15% ethyl acetate in petroleum ether as eluant). First eluted was the 20R-isomer:
NMR: δ = 0.05 (m, 12H), 0.62 (s, 3H), 0.86 (s, 9H), 0.89 (s, 9H), 1.10-2.10 (m, 14H), 1.15 (d, 3H), 2.30 (bd, 1H), 2.53 (dd, 1H), 2.89 (m, 1H), 2.89 (m, 1H), 3.71 (m, 1H), 4.21 (m, 1H), 4.52 (m, 1H), 4.93 (m, 1H), 4.98 (m, 1H), 5.81 (d, 1H), 6.45 (d, 1H) ppm.

The fractions containing the more polar isomer (3) were evaporated to give a colourless residue which was crystallized from methanol.

NMR, δ = 0.052 (bd, 12H), 0.54 (s, 3H), 0.85 (s, 9H), 0.89 (s, 9H), 1.22 (d, 3H), 1.20-2.10 (m, 14H), 2.30 (bd, 1H), 2.55 (dd, 1H), 2.87 (m, 1H), 3.72 (m, 1H), 4.21 (m, 1H), 4.52 (m, 1H), 4.94 (bs, 1H), 4.98 (m, 1H), 5.82 (d, 1H), 6.44 (d, 1H) ppm.

### Preparation 3: Compound 8, via compounds 5, 6 and 7

A solution of compound 4 (3.1 g) in dry THF (30 ml) was treated dropwise with MeLi (1.5 M in ether, 7 ml) with stirring at -40°C. After 10 minutes the reaction mixture was worked up (ether) to give crude 5 as a ca. 2:3 mixture of diastereoisomers [NMR: δ 0.99 (minor isomer) and 1.13 (major isomer), (2d, total 3H, J = 6.5)].

This was dissolved in dichloromethane (75 ml) and pyridinium dichromate (7 g) and TsOH (0.05 g) added. The mixture was stirred overnight and then diluted with ether (200 ml) and filtered through celite. The filtrate was concentrated in vacuo and purified by chromatography (silica gel, 5% ether in petroleum ether as eluant) to give 6 [NMR: δ inter alia 1.07 (d, 3H, J = 7), 2.08 (s, 3H), 2.48 (m, 1H) and 3.99 (m, 1H)].

A solution of 6 (1 g), m-chloroperbenzoic acid (1.2 g) and TsOH (0.05 g) in dichloromethane (40 ml) was heated under reflux for 24 hours. After cooling, the mixture was diluted with ether and extracted consecutively with 5% sodium bicarbonate solution, 10% sodium sulphite solution, 5% sodium bicarbonate, and brine. Drying and concentration in vacuo gave a residue containing 7, which was directly hydrolysed by treatment of a solution in THF (5 ml) and methanol (20 ml) with a KOH solution (4N aqueous, 5 ml) at 50°C for 1 hour. Work-up (dichloromethane) and purification by chromatography (silica gel; 40% ether in petroleum ether as eluant) gave 8 as an oil which crystallized on standing (M.p. 78-80°C).

NMR: δ = -0.02 and 0.00 (each s, 3H), 0.88 (s, 9H), 1.0-1.9 (m, 16H), 1.19 (d, 3H, J = 6), 3.67 and 4.00 (each m, 1H).

### Preparation 4: Compound 9

To a solution of compound 3 (169 mg, 0.3 mmol) in dry tetrahydrofuran (5 ml) potassium hydroxide (0.70 g, 10 mmol), 18-Crown-6 (40 mg) and 2-(6-bromo-2-methyl-2-hexyloxy)-tetrahydro-4H-pyran (Preparation 5) (1.7 g, 6 mmol) were added. The mixture was stirred vigorously overnight. The reaction mixture was filtered, and the filtrate evaporated in vacuo.

The residue was purified by chromatography (silica gel, 5% to 10% ether in petroleum ether as eluant) to give the title compound.

NMR: δ = 0.055 (bs, 12H), 0.51 (s, 3H), 0.86 (s, 9H), 0.89 (s, 9H), 1.14 (d, 3H), 1.17 (s, 3H), 1.19 (s, 3H), 0.90-2.05 (m, 25H), 2.31 (bd, 1H), 2.54 (dd, 1H), 2.85 (m, 1H), 3.20 (m, 2H), 3.41 (m, 1H), 3.56 (m, 1H), 3.95 (m, 1H), 4.21 (m, 1H), 4.52 (m, 1H), 4.71 (m, 1H), 4.93 (bs, 1H), 4.98 (bs, 1H), 5.82 (d, 1H), 6.44 (d, 1H) ppm.

### Preparation 5: 2-(6-Bromo-2-methyl-2-hexyloxy)-tetrahydro-4H-pyran

To a stirred, ice-cooled solution of ethyl 5-bromo-pentanoate (18.7 ml) in dried ether (100 ml) was added dropwise over 1 hour a filtered solution of Grignard reagent, prepared from magnesium (10 g) and methyl iodide 25 ml) in dried ether (200 ml). After a further 30 minutes on the ice-bath, the reaction mixture was allowed to warm to room temperature over 30 minutes before being poured onto a stirred, ice-cooled solution of ammonium chloride (30 g) in water (200 ml). After the vigorous reaction had subsided, the ether layer was separated, and the aqueous layer was extracted with more ether. The combined ether layers were washed consecutively with water and saturated aqueous sodium chloride, dried, and concentrated in vacuo to give the crude intermediate (6-bromo-2-methyl-2-hexanol) as a pale yellow oil. This was dissolved in dichloromethane (100 ml), then 3,4-dihydro-2H-pyran (8.9 ml) and pyridinium p-tolueneaulfonate (0.8 g) were added at room temperature. After 1 hour, the reaction solution was diluted with ether (250 ml) and extracted consecutively with saturated aqueous sodium hydrogen carbonate (150 ml), water (100 ml) and saturated aqueous sodium chloride (100 ml). After drying and removal of the solvent in vacuo, the product was purified by chromatography (150 g silica gel, 10% ether in petroleum ether as eluant) to give the desired compound as a colourless oil.

NMR: δ = 1.20 (s, 3H), 1.22 (s, 3H), 1.40-1.95 (m, 12 H), 3.42 (t, 2H), 3.45 (m, 1H), 4.72 (m, 1H) ppm.

### Preparation 6: Compound 10

The compound was prepared using the procedure of Preparation 4, but replacing the 2-(6-bromo-2-methyl-2--hexyloxy)-tetrahydro-4H-pyran with an equivalent amount of 6-bromo-3-ethyl-3-(trimethylsilyloxy)hexane.

NMR in agreement with formula.

### Preparation 7: Compound 11

The compound was prepared using the procedure of Preparation 4, but replacing the 2-(6-bromo-2-methyl-2-hexyloxy)-tetrahydro-4H-pyran with an equivalent amount of 7-bromo-2-methyl-2-(trimethylsilyloxy)heptane.

NMR in agreement with formula.

### Preparation 8: Compound 15

The compound was prepared using the procedure of Preparation 7, but replacing compound 3 with compound 8 (150 mg).

NMR in agreement with formula.

### Preparation 9: Compound 12

A solution of compound 9 (88 mg), anthracene (60 mg) and triethylamine (2 drops) in dichloromethane (10 ml) under N₂ in a Pyrex flask was irradiated with light from a high pressure ultra-violet lamp, type TQ 150Z2 (Hanau), at room temperature for 55 minutes. The solution was filtered, concentrated in vacuo, and the residue purified by chromatography (silica gel, 10% ether in petroleum ether as eluant) to give the title compound.

NMR: δ = 0.056 (m, 12H), 0.50 (s, 3H), 0.86 (s, 9H), 0.87 (s, 9H), 1.14 (d, 3H), 1.17 (s, 3H), 1.19 (s, 3H), 1.0-2.0 (m, 25H), 2.21 (dd, 1H), 2.43 (m, 1H), 2.82 (m, 1H), 3.20 (m, 2H), 3.42 (m, 1H), 3.55 (m, 1H), 3.93 (m, 1H), 4.18 (m, 1H), 4.36 (m, 1H), 4.70 (m, 1H), 4.85 (bd, 1H), 5.17 (bd, 1H), 6.01 (d, 1H), 6.21 (d, 1H) ppm.

### Preparation 10: Compound 13

The compound was prepared using the procedure of Preparation 9, but substituting compound 9 with compound 10.

NMR in agreement with structure.

### Preparation 11: Compound 14

The compound was prepared using the procedure of Preparation 9, but substituting compound 9 with compound 11.

NMR in agreement with structure.

### Preparation 12: Compound 14 (Alternative method)

A stirred solution of Compound 15 (90 mg) in ethyl acetate (0.5 ml) was treated with a mixture of acetonitrile (3.5 ml) and 40% aqueous hydrofluoric acid (0.5 ml). After 3 hours, the mixture was rendered basic with 2N sodium hydroxide solution and worked up (ethyl acetate) to give the crude intermediate desilylated product as an oil. This was dissolved in dichloromethane (4 ml) and pyridinium chlorochromate (0.2 g) was added. The mixture was stirred for 90 minutes and then diluted with ether and filtered through celite. The filtrate was concentrated in vacuo to give the crude intermediate VIII (R³ = 6-methyl-6-hydroxy-1-heptyl) as an oil. This was dissolved in dry THF and treated with trimethylsilylimidazole (150 mg). After stirring for 3 hours, the mixture was partially concentrated in vacuo, and the residue was purified by chromatography (silica gel, 20% ether in petroleum ether as eluant) to give the intermediate VIII (R³ = 6-methyl-6-(trimethylsilyloxy)-1-heptyl) as an oil. A solution of this (60 mg) in dry THF (2 ml) was added at -70°C under N₂ to a preformed solution of the lithio-derivative of IX. [This was prepared by adding n-BuLi (1.3 M in hexanes, 0.15 ml) to a solution of compound IX (110 mg) in dry THF (2 ml) at -70°C under N₂ and stirring at this temperature for 15 minutes]. After 1 hour, the reaction mixture was worked up (ether) and the residue purified by chromatography (silica gel, 5% ether in petroleum ether as eluant) to give the title compound, identifical to material prepared in Preparation 11.

### Example 1: 1(S),3(R)-Dihydroxy-20(S)-(5-hydroxy-5-methyl-1-hexyloxy)-9,10-seco-pregna-5(Z),7(E),10(19)-triene (Compound 101)

Compound 12 (50 mg) was dissolved in a mixture of acetonitrile (5 ml) and ethyl acetate (1 ml). A 5% solution of HF in acetonitrile/water 8:1 (2 ml) was added, and the solution was stirred under N₂ for 40 minutes. Ethyl acetate (50 ml) was added, and the mixture was extracted with saturated aqueous sodium bicarbonate (10 ml) and water (10 ml), dried and concentrated in vacuo. The residue was purified by chromatography (silica gel, ethyl acetate as eluant) to give the title compound.

NMR: δ = 0.53 (s, 3H), 1.15 (d, 3H), 1.21 (s, 6H), 1.2-2.1 (m, 22H), 2.32 (dd, 1H), 2.60 (dd, 1H), 2.82 (dd, 1H), 3.21 (m, 2H), 3.56 (m, 1H), 4.22 (m, 1H), 4.43 (m, 1H), 4.99 (bs, 1H), 5.33 (m, 1H), 6.02 (d, 1H), 6.37 (d, 1H) ppm.

### Example 2: 1(S),3(R)-Dihydroxy-20(S)-(4-hydroxy-4-ethyl-1-hexyloxy)-9,10-seco-pregna-5(Z),7(E),10(19)-triene (Compound 102)

The compound was prepared using the procedure of Example 1, but substituting compound 13 for compound 12.

NMR in agreement with structure.

### Example 3: 1(S),3(R)-Dihydroxy-20(S)-(6'-hydroxy-6'-methyl-1'-heptyloxy-9,10-seco-pregna-5(Z),7(E),10(19)-triene (Compound 103)

The compound was prepared using the procedure of Example 1, but substituting compound 14 for compound 12.

NMR in agreement with structure.

### Example 4 Capsules containing Compound 101

101 was dissolved in arachis oil to a final concentration of 10 µg 101/ml oil. 10 Parts by weight of gelatine, 5 parts by weight glycerine, 0.08 parts by weight potassium sorbate, and 14 parts by weight distilled water were mixed together with heating and formed into soft gelatine capsules. These were then filled each with 100 µl of the 101 in oil solution, such that each capsule contained 1.0 µg 101.

### Example 5 Dermatological Cream Containing Compound 101

In 1 g almond oil was dissolved 0.5 mg 101. To this solution was added 40 g of mineral oil and 20 g of self-emulsifying beeswax. The mixture was heated to liquify. After the addition of 40 ml hot water, the mixture was mixed well. The resulting cream contains approximately 5 µg of 101 per gram of cream.

## Claims (Claims for the following Contracting State(s): AT, BE, CH, DE, DK, FR, GB, IT, LI, LU, NL, SE)

1. A compound of the formula I 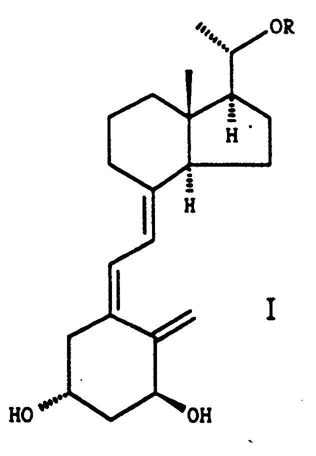 in which formula R stands for an alkyl group containing from 7 to 12 carbon atoms optionally substituted with a hydroxy group; and derivatives of the compounds of formula I in which one or more hydroxy groups have been transformed into -O-acyl or -O-glycosyl or phosphate ester groups, such masked groups being hydrolyzable in vivo.

2. A compound according to claim 1, in which R is a group of formula II 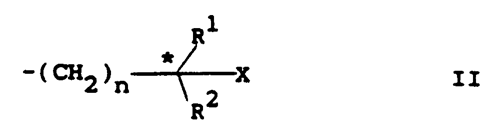 where n is an integer from 1 to 7; R¹ and R², which may be the same or different, stand for hydrogen, C₁-C₅-alkyl, C₃-C₇-cycloalkyl, or taken together with the carbon atom (starred in formula II) bearing the group X, R¹ and R² can form a C₃-C₈ carbocyclic ring; and X stands for hydroxy or hydrogen.

3. A diastereoisomer of a compound according to claims 1 or 2, in pure form; or a mixture of diastereoisomers of a compound according to claim 1 or 2.

4. A compound according to claim 1 which is 1(S),3(R)-dihydroxy-20(S)-(5'-hydroxy-5'-methyl-1'-hexyloxy)-9,10-secopregna-5(Z),7(E),10(19)-triene.

5. A method for producing a compound of formula I of claim 1 or an analogue thereof using the following steps:
a) 1(S),3(R)-bis-(tert-butyldimethylsilyloxy)-20(S)-formyl-9,10-seco-pregna-5(E),7(E),10(19)-triene is oxidized with oxygen in the presence of a basic catalyst, to form 1(S),3(R)-bis-(tert-butyldimethylsilyloxy)-9,10-seco-pregna-5(E),7(E),10(19)-triene-20-one;
b) 1(S),3(R)-bis-(tert-butyldimethylsilyloxy)-9,10-seco-pregna-5(E),7(E),10(19)-triene-20-one is reduced, e.g. by sodium borohydride, to form 1(S),3(R)-bis-(tert-butyldimethylsilyloxy)-9,10-seco-pregna-5(E),7(E),10(19)-triene-20(S)-ol;
c) 1(S),3(R)-bis-(tert-butyldimethylsilyloxy)-9,10-seco-pregna-5(E),7(E),10(19)-triene-20(S)-ol is alkylated under basic conditions with a side chain building block of formula Z-R³, in which Z is a leaving group such as halogen, p-toluenesulphonyloxy or methanesulphonyloxy, to form a compound of formula III 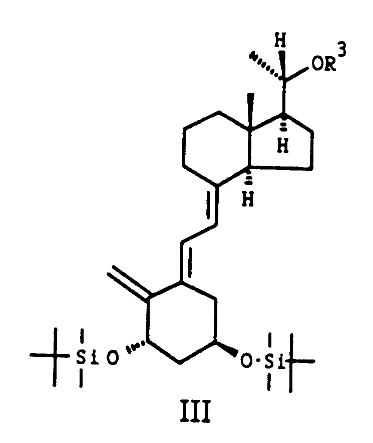 in which R³ is R (R is as defined above or an analogue thereof) or optionally a radical which can be converted to R;
d) a compound of the above formula III is subjected to a triplet-sensitized photoisomerisation, and, if necessary, conversion of R³ to R, and deprotection, to form the desired compound of formula I of claim 1 or an analogue thereof.

6. A method for producing a compound of formula I of claim 1 or an analogue thereof using the following steps:
a) 1(S)-[1'(S)-formylethyl]-7a(S)-methyl-3a(R)-octahydro-4(R)-[(tert-butyldimethylsilyl)oxy]-1H-indene or a similar alcohol-protected analogue is converted to 1(S)-[1ʼ(S)-hydroxy-ethyl]-7a(S)-methyl-3a(R)-octahydro-4(R)-[(tert-butyldimethylsilyl)oxy]-1H-indene or a similar alcohol-protected analogue, for example by the sequence of reaction with methyl-lithium, oxidation, Baeyer-Villiger reaction, and saponification;
b) 1(S)-[1'(S)-hydroxy-ethyl]-7a(S)-methyl-3a(R)-octahydro-4(R)-[(tert-butyldimethylsilyl)oxy]-1H-indene or a similar alcohol-protected analogue is alkylated under basic conditions with a side chain building block of formula Z-R³, in which Z is a leaving group such as halogen, p-toluenesulphonyloxy or methanesulphonyloxy, and in which R³ is R (R is as defined above or an analogue thereof) or optionally a radical, which can be converted to R, to form a compound of formula VII 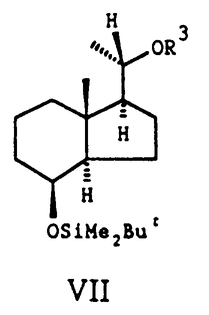 followed by conversion of the protected alcohol group to a keto group via deprotection and oxidation, to give a compound of formula VIII 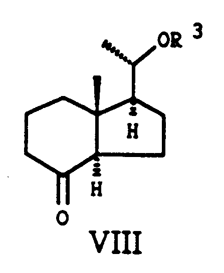
c) Reaction of a compound of formula VIII with the anion derived from [2-[3(S),5(R)-bis-(tert-butyldimethylsilyloxy)-2-methylene-cyclohexyl-(Z)-idene]ethyl]diphenylphosphine oxide, followed by deprotection of the alcohol groups, and, if necessary, conversion of R³ to R or an analogue thereof, to form the desired compound of formula I of claim 1 or an analogue thereof.

7. Intermediate for the synthesis of compounds of formula I and analogues thereof which is
1(S),3(R)-bis(tert-butyldimethylsilyloxy)-9,10-seco-pregna-5(E),7(E),10(19)-triene-20-(S)-ol
or 1(S)-[1'(S)-hydroxyethyl]-7a(S)-methyl-3a(R)-octahydro-4(R)-[(tert-butyldimethylsilyl)oxy]-1H-indene.

8. A pharmaceutical composition containing an effective amount of one or more of the compounds of claim 1, together with pharmaceutically acceptable, non-toxic carriers and/or auxiliary agents.

9. A pharmaceutical composition according to claim 8 in dosage unit form.

10. A dosage unit according to claim 9 containing from 0.5 - 500 µg, preferably from 1 - 250 µg of a compound of formula I.

11. The use of a compound of any one of claims 1 to 4 for preparing a pharmaceutical composition for the treatment and prophylaxis of autoimmune diseases, including diabetes mellitus, hypertension, inflammatory diseases such as rheumatoid arthritis and asthma as well as diseases characterized by abnormal cell differentiation and/or cell proliferation, and/or imbalance in the immune system.

12. The use according to claim 11 for the treatment or prophylaxis of cancer.

13. The use according to claim 11 for the treatment of psoriasis.

## Claims (Claims for the following Contracting State(s): ES)

1. A method for producing a compound of the formula I 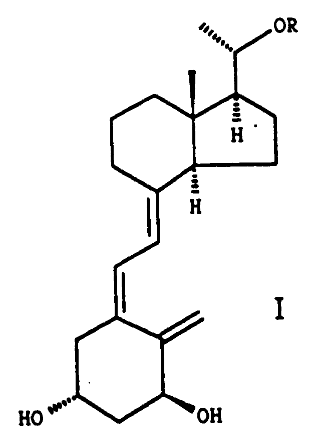 in which formula R stands for an alkyl group containing from 7 to 12 carbon atoms optionally substituted with a hydroxy group; and derivatives of the compounds of formula I in which one or more hydroxy groups have been transformed into -O-acyl or -O-glycosyl or phosphate ester groups, such masked groups being hydrolyzable in vivo, using the following steps:
a) 1(S),3(R)-bis-(tert-butyldimethylsilyloxy)-20(S)-formyl-9,10-seco-pregna-5(E),7(E),10(19)-triene is oxidized with oxygen in the presence of a basic catalyst, to form 1(S),3(R)-bis-(tert-butyldimethylsilyloxy)-9,10-seco-pregna-5(E),7(E),10(19)-triene-20-one;
b) 1(S),3(R)-bis-(tert-butyldimethylsilyloxy)-9,10-seco-pregna-5(E),7(E),10(19)-triene-20-one is reduced, e.g. by sodium borohydride, to form 1(S),3(R)-bis-(tert-butyldimethylsilyloxy)-9,10-seco-pregna-5(E),7(E),10(19)-triene-20(S)-ol;
c) 1(S),3(R)-bis-(tert-butyldimethylsilyloxy)-9,10-seco-pregna-5(E),7(E),10(19)-triene-20(S)-ol is alkylated under basic conditions with a side chain building block of formula Z-R³, in which Z is a leaving group such as halogen, p-toluenesulphonyloxy or methanesulphonyloxy, to form a compound of formula III 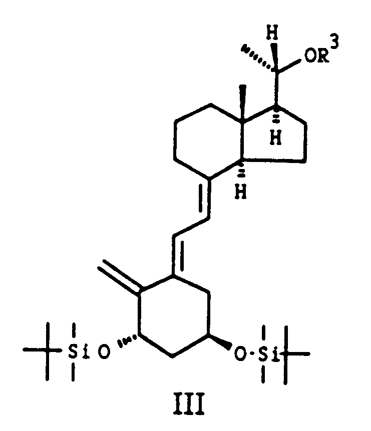 in which R³ is R (R is as defined above or an analogue thereof) or optionally a radical which can be converted to R;
d) a compound of the above formula III is subjected to a triplet-sensitized photoisomerisation, and, if necessary, conversion of R³ to R, and deprotection, to form the desired compound of formula I or an analogue thereof,
or using the following steps:
a) 1(S)-[1'(S)-formylethyl]-7a(S)-methyl-3a(R)-octahydro-4(R)-[(tert-butyldimethylsilyl)oxy]-1H-indene or a similar alcohol-protected analogue is converted to 1(S)-[1'(S)-hydroxy-ethyl]-7a(S)-methyl-3a(R)-octahydro-4(R)-[(tert-butyldimethylsilyl)oxy]-1H-indene or a similar alcohol-protected analogue, for example by the sequence of reaction with methyl-lithium, oxidation, Baeyer-Villiger reaction, and saponification.
b) 1(S)-[1'(S)-hydroxy-ethyl]-7a(S)-methyl-3a(R)-octahydro-4(R)-[(tert-butyldimethylsilyl)oxy]-1H-indene or a similar alcohol-protected analogue is alkylated under basic conditions with a side chain building block of formula Z-R³, in which Z is a leaving group such as halogen, p-toluenesulphonyloxy or methanesulphonyloxy, and in which R³ is R (R is as defined above or an analogue thereof) or optionally a radical, which can be converted to R, to form a compound of formula VII 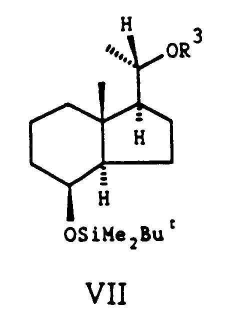 followed by conversion of the protected alcohol group to a keto group via deprotection and oxidation, to give a compound of formula VIII 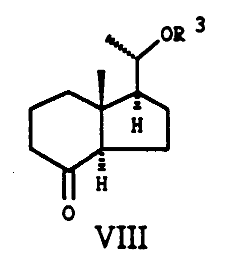
c) Reaction of a compound of formula VIII with the anion derived from [2-[3(S),5(R)-bis-(tert-butyldimethylsilyloxy)-2-methylene-cyclohexyl-(Z)-idene]ethyl]diphenylphosphine oxide, followed by deprotection of the alcohol groups, and, if necessary, conversion of R³ to R or an analogue thereof, to form the desired compound of formula I or an analogue thereof.

2. A method according to claim 1, in which R is a group of formula II 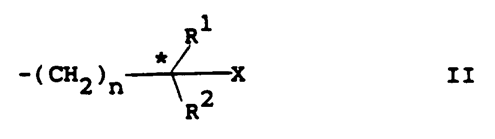 where n is an integer from 1 to 7; R¹ and R², which may be the same or different, stand for hydrogen, C₁-c₅-alkyl, C₃-C₇-cycloalkyl, or taken together with the carbon atom (starred in formula II) bearing the group X, R¹ and R² can form a C₃-C₈ carbocyclic ring; and X stands for hydroxy or hydrogen.

3. A method according to claim 1 or 2 wherein a diastereoisomer of a compound according to claim 1 or 2, in pure form, or a mixture of diastereoisomers of a compound according to claim 1 or 2 is prepared.

4. A method according to claim 1 in which 1(S),3(R)-dihydroxy-20(S)-(5'-hydroxy-5'methyl-1'-hexyloxy)-9,10-secopregna-5(Z),7(E),10(19)-triene is prepared.

5. A method for producing the intermediate for the synthesis of compounds of formula I and analogues thereof, which is
1(S),3(R)-bis(tert-butyldimethylsilyloxy)-9,10-seco-pregna-5(E),7(E),10(19)-triene-20-(S)-ol,
in which
a) 1(S),3(R)-bis-(tert-butyldimethylsilyloxy)-20(S)-formyl-9,10-seco-pregna-5(E),7(E),10(19)-triene is oxidized with oxygen in the presence of a basic catalyst, to form 1(S),3(R)-bis-(tert-butyldimethylsilyloxy)-9,10-seco-pregna-5(E),7(E),10(19)-triene-20-one; and
b) 1(S),3(R)-bis-(tert-butyldimethylsilyloxy)-9,10-seco-pregna-5(E),7(E),10(19)-triene-20-one is reduced, e.g. by sodium borohydride, to form 1(S),3(R)-bis-(tert-butyldimethylsilyloxy)-9,10-seco-pregna-5(E),7(E),10(19)-triene-20(S)-ol;

6. A method for producing the intermediate for the synthesis of compounds of formula I or analogues thereof, which is
1(S)-[1'(S)-hydroxyethyl]-7a(S)-methyl-3a(R)-octahydro-4(R)-[(tert-butyldimethylsilyl)oxy]-1H-indene,
wherein 1(S)-[1'(S)-formylethyl]-7a(S)-methyl-3a(R)-octahydro-4(R)-[(tert-butyldimethylsilyl)oxy]-1H-indene or a similar alcohol-protected analogue is converted to the desired compound, for example by the sequence of reaction with methyl-lithium, oxidation, Baeyer-Villiger reaction, and saponification.

7. A method for producing a pharmaceutical composition which comprises incorporating an effective amount of one or more of the compounds as defined in claim 1, into pharmaceutically acceptable, non-toxic carriers and/or auxiliary agents.

8. A method according to claim 7 wherein the composition is prepared in dosage unit form.

9. A method according to claim 8, wherein the dosage unit contains from 0.5 - 500 µg, preferably from 1 - 250 µg of a compound of formula I.

## Patentansprüche (Patentansprüche für folgende(n) Vertragsstaat(en): AT, BE, CH, DE, DK, FR, GB, IT, LI, LU, NL, SE)

1. Verbindung der Formel I 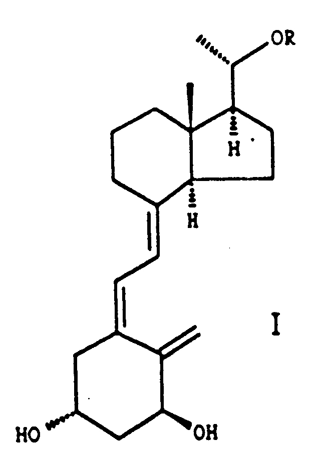 worin R für eine Alkylgruppe mit 7 bis 12 Kohlenstoffatomen, die gegebenenfalls durch eine Hydroxygruppe substituiert ist, steht; und die Derivate der Verbindungen der Formel I, bei denen eine oder mehrere Hydroxygruppen in -O-Acyl- oder O-Glycosyl- oder Phosphatestergruppen überführt worden sind, wobei diese maskierten Gruppen in vivo hydrolysierbar sind.

2. Verbindung nach Anspruch 1, wobei R für eine Gruppe der Formel II 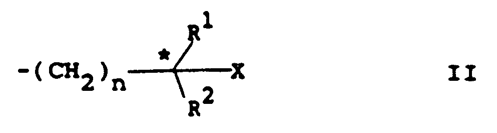 steht, worin n für eine ganze Zahl von 1 bis 7 steht; R¹ and R², die gleich oder verschieden sind, für Wasserstoff, C₁-C₅-Alkyl, C₃-C₇-Cycloalkyl stehen oder worin R¹ und R² zusammen mit dem die Gruppe X aufweisenden Kohlenstoffatom (in Formel II mit einem Stern gekennzeichnet) einen carbocyclischen C₃-C₈-Ring bilden; und X für Wasserstoff oder Hydroxy steht.

3. Diastereomer einer Verbindung nach Anspruch 1 oder 2 in reiner Form oder eine Mischung von Diastereomeren einer Verbindung nach Anspruch 1 oder 2.

4. Verbindung nach Anspruch 1, nämlich 1(S),3(R)-Dihydroxy-20(S)-(5'-hydroxy-5'-methyl-1'-hexyloxy)-9,10-secopregna-5(Z),7(E),10(19)-trien.

5. Verfahren zur Herstellung einer Verbindung der Formel I nach Anspruch 1 unter Verwendung der folgenden Stufen, wobei
a) 1(S),3(R)-Bis-(tert-butyldimethylsilyloxy)-20(S)formyl-9,10-seco-pregna-5(E),7(E),10(19)-trien mit Sauerstoff in Anwesenheit eines basischen Katalysators zu 1(S),3(R)-Bis-(tert-butyldimethylsilyloxy)-9,10-seco-pregna-5(E),7(E),10(19)-trien-20-on oxydiert wird;
b) 1(S),3(R)-Bis-(tert-butyldimethylsilyloxy)-9,10-seco-pregna-5(E),7(E),10(19)-trien-20-on, beispielsweise mit Natriumborhydrid, zu 1(S),3(R)-Bis-(tert-butyldimethylsilyloxy)-9,10-seco-pregna-5(E),7(E),10(19)-trien-20((S)-ol reduziert wird;
c) 1(S),3(R)-Bis-(tert-butyldimethylsilyloxy)-9,10-seco-pregna-5(E),7(E),10(19)-trien-20(S)-ol unter basischen Bedingungen mit einem Seitenketten bildenden Block der Formel Z-R³, worin Z eine Abgangsgruppe bedeutet, wie Halogen, p-Toluolsulfonyloxy oder Methansulfonyloxy, zu einer Verbindung der Formel III 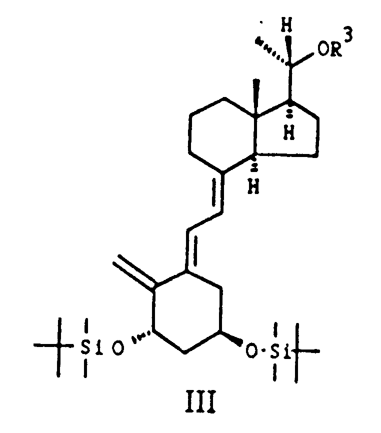 worin R³ für R (R ist wie oben definiert oder ein Analogon davon) oder gegebenenfalls einen Rest steht, der in R umgewandelt werden kann, alkyliert wird;
d) eine Verbindung der obigen Formel III einer triplettsensibilisierten Photoisomerisierung und, falls erforderlich, R³ in R umgewandelt wird, und einer Deprotektion unterworfen wird, um die gewünschte Verbindung der Formel I nach Anspruch 1 oder ein Analogon davon zu erhalten.

6. Verfahren zur Herstellung einer Verbindung der Formel I nach Anspruch 1 oder eines Anlogon davon unter Verwendung der folgenden Stufen, wobei:
a) 1(S)-[1'(S)-Formylethyl]-7a(S)-methyl-3a(R)-octahydro-4(R)-[(tert-butyldimethylsilyl)oxy]-1H-inden oder ein ähnliches alkoholgeschütztes Analogon davon zu 1(S)-[1'(S)-hydroxyethyl]-7a(S)-methyl-3a(R)-octahydro-4(R)-[(tert-butyldimethylsilyl)oxy]-1H-inden oder einem ähnlichen alkoholgeschützten Analogon davon umgesetzt wird, beispielsweise durch die Reaktionsfolge mit Methyllithium, Oxidation, Baeyer-Villiger-Reaktion und Verseifung;
b) 1(S)-[1'(S)-Hydroxy-ethyl]-7a(S)-methyl-3a(R)-octahydro-4(R)-[(tertbutyldimethylsilyl)oxy]-1H-inden oder ein ähnliches alkoholgeschütztes Analogon unter basischen Bedingungen mit einem Seitenketten bildenden Block der Formel Z-R³, worin Z für eine Abgangsgruppe, wie Halogen, p-Toluolsulfonyloxy oder Methansulfonyloxy, steht und worin R³ für R (wobei R die oben angegebenen Bedeutungen besitzt oder ein Analogon davon bedeutet) oder gegebenenfalls einen Rest steht, der in R umgewandelt werden kann, zu einer Verbindung der Formel VII 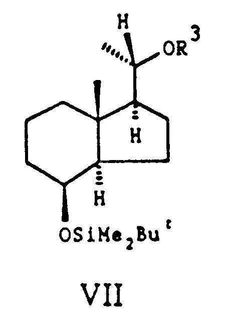 alkyliert wird, und anschließend die geschützte Alkoholgruppe durch Deprotektion und Oxidation in eine Ketogruppe umgewandelt wird, wobei eine Verbindung der Formel VIII 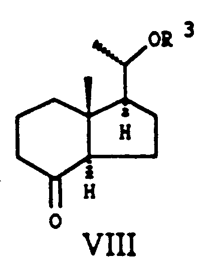 erhalten wird,
c) eine Verbindung der Formel VIII mit dem Anion von [2-[3(S),5(R)-Bis-(tert-butyldimethylsilyloxy)-2-methylencyclohexyl-(Z)-iden]ethyl]diphenylphospinoxid umgesetzt wird, anschließend die Alkoholgruppen entschützt werden und, falls erforderlich, R³ in R oder ein Analogon davon umgewandelt wird, um die gewünschte Verbindung der Formel I nach Anspruch 1 oder ein Analogon davon zu bilden.

7. Zwischenprodukt für die Synthese der Verbindungen der Formel I und der Analoga davon, nämlich
1(S),3(R)-Bis(tert-butyldimethylsilyloxy)-9,10-seco-pregna-5(E),7(E),10(19)-trien-20(S)-ol oder
1(S)-[1'(S)-hydroxyethyl]-7a(S)-methyl-3a(R)-octahydro-4(R)-[(tert-butyldimethylsilyl(oxy]-1H-inden.

8. Pharmazeutisches Mittel enthaltend eine wirksame Menge einer oder mehrerer Verbindungen nach Anspruch 1, zusammen mit pharmazeutisch akzeptablen, nicht-toxischen Trägern und/oder Hilfsmitteln.

9. Pharmazeintisches Mittel nach Anspruch 8 in Form einer Dosiseinheit.

10. Dosiseinheit nach Anspruch 9, enthaltend 0,5 - 500 µg, vorzugsweise 1 - 250 µg einer Verbindung der Formel I.

11. Verwendung der Verbindungen nach einem der Ansprüche 1 bis 4 zur Herstellung eines pharmazeutischen Mittels zur Behandlung und Prophylaxe von Autoimmunerkrankungen, einschließlich Diabetis mellitus, Hypertension, inflammatorischen Erkrankungen, wie rheumatoide Arthritis und Asthma sowie Erkrankungen, die durch eine abnormale Zelldifferenzierung und/oder Zellproliferation und/oder ein gestörtes Gleichgewicht im Immunsystem charakterisiert sind.

12. Verwendung nach Anspruch 11 zur Behandlung oder Prophylaxe eines Karzinoms.

13. Verwendung nach Anspruch 11 zur Behandlung von Psoriasis.

## Patentansprüche (Patentansprüche für folgende(n) Vertragsstaat(en): ES)

1. Verfahren zur Herstellung einer Verbindung der Formel I 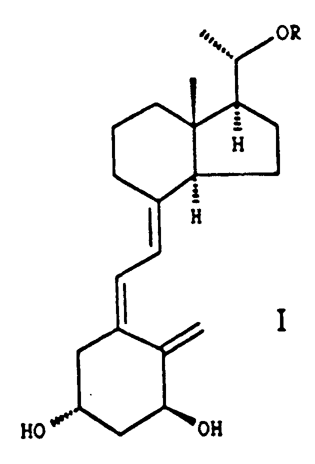 worin R für eine Alkylgruppe mit 7 bis 12 Kohlenstoffatomen, die gegebenenfalls durch eine Hydroxygruppe substituiert ist, steht; und die Derivate der Verbindungen der Formel I, bei denen eine oder mehrere Hydroxygruppen in -O-Acyl- oder O-Glycosyl- oder Phosphatestergruppen überführt worden sind, wobei diese maskierten Gruppen in vivo hydrolysierbar sind, unter Verwendung der folgenden Stufen, wobei
a) 1(S),3(R)-Bis-(tert-butyldimethylsilyloxy)-20(S)-formyl-9,10-seco-pregna-5(E),7(E),10(19)-trien mit Sauerstoff in Anwesenheit eines basischen Katalysators zu 1(S),3(R)-Bis-(tert-butyldimethylsilyloxy)-9,10-seco-pregna-5(E),7(E),10(19)-trien-20-on oxydiert wird;
b) 1(S),3(R)-Bis-(tert-butyldimethylsilyloxy)-9,10-seco-pregna-5(E),7(E),10(19)-trien-20-on, beispielsweise mit Natriumborhydrid, zu 1(S),3(R)-Bis-(tert-butyldimethylsilyloxy)-9,10-seco-pregna-5(E),7(E),10(19)-trien-20((S)-ol reduziert wird;
c) 1(S),3(R)-Bis-(tert-butyldimethylsilyloxy)-9,10-segna-pregna-5(E),7(E),10(19)-trien-20(S)-ol unter basischen Bedingungen mit einem Seitenketten bildenden Block der Formel Z-R³, worin Z eine Abgangsgruppe bedeutet, wie Halogen, p-Toluolsulfonyloxy oder Methansulfonyloxy, zu einer Verbindung der Formel III 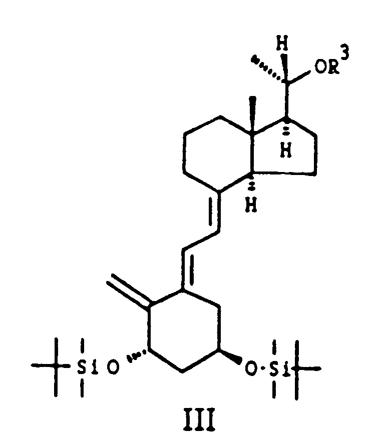 worin R³ für R (R ist wie oben definiert oder ein Analogon davon) oder gegebenenfalls einen Rest steht, der in R umgewandelt werden kann, alkyliert wird;
d) eine Verbindung der obigen Formel III einer triplettsensibilisierten Photoisomerisierung und, falls erforderlich, R³ in R umgewandelt wird, und einer Deprotektion unterworfen wird, um die gewünschte Verbindung der Formel I oder ein Analogon davon zu erhalten
oder unter Verwendung der folgenden Stufen, wobei:
a) 1(S)-[1'(S)-Formylethyl]-7a(S)-methyl-3a(R)-octahydro-4(R)-[(tert-butyldimethylsilyl)oxy]-1H-inden oder ein ähnliches alkoholgeschütztes Analogon davon zu 1(S)-[1'(S)-hydroxyethyl]-7a(S)-methyl-3a(R)-octahydro-4(R)-[(tert-butyldimethylsilyl)oxy]-1H-inden oder einem ähnlichen alkoholgeschützten Analogon davon umgesetzt wird, beispielsweise durch die Reaktionsfolge mit Methyllithium, Oxidation, Baeyer-Villiger-Reaktion und Verseifung;
b) 1(S)-[1'(S)-Hydroxy-ethyl]-7a(S)-methyl-3a(R)-octahydro-4(R)-[(tertbutyldimethylsilyl)oxy]-1H-inden oder ein ähnliches alkoholgeschütztes Analogon unter basischen Bedingungen mit einem Seitenketten bildenden Block der Formel Z-R³, worin Z für eine Abgangsgruppe, wie Halogen, p-Toluolsulfonyloxy oder Methansulfonyloxy, steht und worin R³ für R (wobei R die oben angegebenen Bedeutungen besitzt oder ein Analogon davon bedeutet) oder gegebenenfalls einen Rest steht, der in R umgewandelt werden kann, zu einer Verbindung der Formel VII 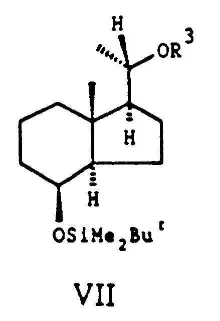 alkyliert wird, und anschließend die geschützte Alkoholgruppe durch Deprotektion und Oxidation in eine Ketogruppe umgewandelt wird, wobei eine Verbindung der Formel VIII 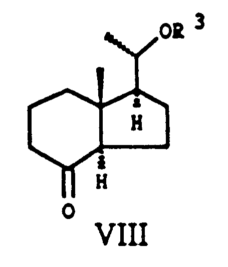 erhalten wird,
c) eine Verbindung der Formel VIII mit dem Anion von [2-[3(S),5(R)-Bis-(tert-butyldimethylsilyloxy)-2-methylencyclohexyl-(Z)-iden]ethyl]diphenylphospinoxid umgesetzt wird, anschließend die Alkoholgruppen entschützt werden und, falls erforderlich, R³ in R oder ein Analogon davon umgewandelt wird, um die gewünschte Verbindung der Formel I oder ein Analogon davon zu bilden.

2. Verfahren nach Anspruch 1, wobei R für eine Gruppe der Formel II 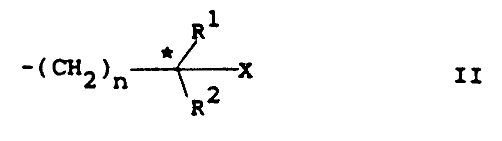 steht, worin n für eine ganze Zahl von 1 bis 7 steht; R¹ and R², die gleich oder verschieden sind, für Wasserstoff, C₁-C₅-Alkyl, C₃-C₇-Cycloalkyl stehen oder worin R¹ und R² zusammen mit dem die Gruppe X aufweisenden Kohlenstoffatom (in Formel II mit einem Stern gekennzeichnet) einen carbocyclischen C₃-C₈-Ring bilden; und X für Wasserstoff oder Hydroxy steht.

3. Verfahren nach Anspruch 1 oder 2, wobei ein Diasteromer einer Verbindung nach Anspruch 1 oder 2 in reiner Form oder eine Mischung von Diastereomeren einer Verbindung nach Anspruch 1 oder 2 hergestellt wird.

4. Verfahren nach Anspruch 1, wobei 1(S),3(R)-Dihydroxy-20(S)-(5,-hydroxy-5'-methyl-1'-hexyloxy)-9,10-secopregna-5(Z),7(E),10(19)-trien hergestellt wird.

5. Verfahren zur Herstellung eines Zwischenproduktes für die Synthese der Verbindungen der Formel I und der Analoga davon, nämlich
1(S),3(R)-Bis(tert-butyldimethylsilyloxy)-9,10-seco-pregna-5(E),7(E),10(19)-trien-20(S)-ol,
wobei
a) 1(S),3(R)-Bis-(tert-butyldimethylsilyloxy)-20(S)formyl-9,10-seco-pregna-5(E),7(E),10(19)-trien mit Sauerstoff in Anwesenheit eines basischen Katalysators zu 1(S),3(R)-Bis-(tert-butyldimethylsilyloxy)-9,10-seco-pregna-5(E),7(E),10(19)-trien-20-on oxydiert wird;
b) 1(S),3(R)-Bis-(tert-butyldimethylsilyloxy)-9,10-seco-pregna-5(E),7(E),10(19)-trien-20-on, beispielsweise mit Natriumborhydrid, zu 1(S),3(R)-Bis-(tert-butyldimethylsilyloxy)-9,10-seco-pregna-5(E),7(E),10(19)-trien-20((S)-ol reduziert wird.

6. Verfahren zur Herstellung des Zwischenproduktes für die Synthese der Verbindungen der Formel I oder der Analoga davon, nämlich
1(S)-[1'(S)-Hydroxyethyl]-7a(S)-methyl)-3a(R)-octahydro-4(R)-[(tert-butyldimethylsilyl)oxy]-1H-inden,
worbei man
1(S(-[1'(S)-Formylethyl]-7a(S)-methyl-3a(R)-octahydro-4(R)-[tert-butyldimethylsilyl)oxy]-1H-inden
oder ein ähnliches alkoholgeschütztes Analogon davon in die gewünschte Verbindung umgewandelt wird, beispielsweise durch die Reaktionsfolge mit Methyllithium, Oxidation, Baeyer-Villiger-Reaktion und Verseifung.

7. Verfahren zur Herstellung eines pharmazeutischen Mittels, wobei man eine wirksame Menge einer oder mehrerer Verbindungen, wie in Anspruch 1 definiert, in pharmazeutisch verträglichen, nicht-toxischen Trägern und/oder Hilfsmitteln aufnimmt.

8. Verfahren nach Anspruch 7, wobei das Mittel in Form einer Dosiseinheit hergestellt wird.

9. Verfahren nach Anspruch 8, wobei die Dosiseinheit 0,5 - 500 µg, vorzugsweise 1 - 250 µg einer Verbindung der Formel I enthält.

## Revendications (Revendications pour l'(les) Etat(s) contractant(s) suivant(s): AT, BE, CH, DE, DK, FR, GB, IT, LI, LU, NL, SE)

1. Un composé de formule I 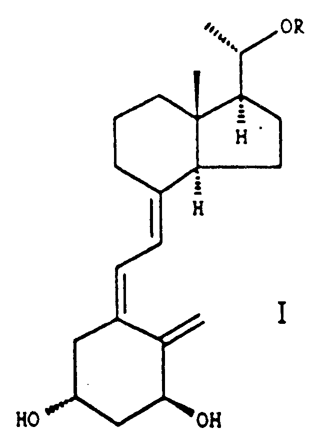 dans laquelle R désigne un groupe alkyle contenant de 7 à 12 atomes de carbone, éventuellement substitué par un groupe hydroxy ; et les dérivés des composés de formule I dans lesquels un ou plusieurs groupes hydroxy ont été transformés en groupes -O-acyle ou O-glycosyle ou ester phosphate, ces groupes masqués étant hydrolysables in vivo.

2. Un composé selon la revendication 1, selon laquelle R est un groupe de formule II 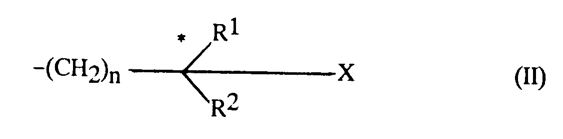 dans laquelle n est un nombre entier de 1 à 7 ; R¹ et R² qui peuvent être identiques ou différents désignent un atome d'hydrogène, un groupe C₁-C₅-alkyle, C₃-C₇-cycloalkyle, ou bien pris ensemble avec l'atome de carbone (marqué d'une étoile dans la formule II) portant le groupe X, R¹ et R² peuvent former un noyau C₃-C₈-carbocyclique; et X désigne un groupe hydroxy ou un atome d'hydrogène.

3. Un diastéréoisomère d'un composé selon la revendication 1 ou 2 sous forme pure ; ou d'un mélange de diastéréoisomères d'un composé selon la revendication 1 ou 2.

4. Un composé selon la revendication 1 qui est le 1(S),3(R)-dihydroxy-20(S)-(5'-hydroxy-5'-méthyl-1'-hexyloxy)-9,10-sccoprégna-5(Z),7(E),10(19)-triène.

5. Un procédé de production d'un composé de formule I selon la revendication 1 ou d'un analogue de ce dernier utilisant les étapes suivantes :
a) le 1(S),3(R)-bis-(tert-butyldiméthylsilyloxy)-20(S)-formyl-9,10-seco-prégna-5(E),7(E),10(19)-triène est oxydé par l'oxygène en présence d'un catalyseur basique, pour former la 1(S),3(R)-bis-(tert-butyldiméthylsilyloxy)-9,10-seco-prégna-5(E),7(E),10(19)-triène-20-one;
b) la 1(S),3(R)-bis-(tert-butyldiméthylsilyloxy)-9,10-secoprégna-5(E),7(E), 10(19)-triène-20-one est réduite, par exemple par le borohydrure de sodium, pour former le 1(S),3(R)-bis-(tert-butyldiméthylsilyloxy)-9,10-seeoprégna-5(E),7(E), 10(19)-triène-20(S)-ol;
c) le 1(S),3(R)-bis-(tert-butyldiméthylsilyloxy)-9,10-secoprégna-5(E),7(E), 10(19)-triène-20(S)-ol est alkylé dans des conditions basiques avec un bloc pour la construction de la chaîne latérale de formule Z-R³, dans laquelle Z est un groupe séparable tel qu'un atome d'halogène, un groupe p-toluènesulfonyloxy ou méthanesulfonyloxy pour former un composé de formule III 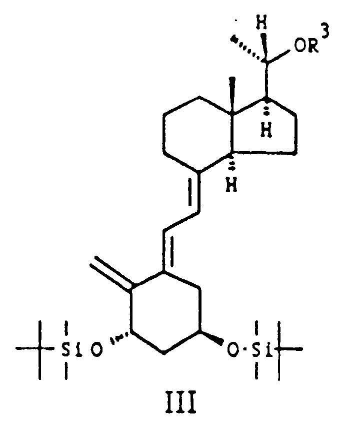 dans laquelle R³ est R (R est comme défini précédemment ou un analogue) ou éventuellement un radical qui peut être converti en R;
d) un composé de formule III précité est soumis à une photoisomérisation avec triplet sansibilisé et, si nécessaire, à une conversion de R³ en R, et une déprotection, pour former le composé désiré de formule I de la revendication 1 ou un analogue de ce dernier.

6. Un procédé de production d'un composé de formule I selon la revendication 1 ou d'un analogue de celui-ci utilisant les étapes suivantes :
a) le 1(S)-[1'(S)-formlyléthyl]7a(S)-méthyl-3a(R)-octahydro-4(R)-[(tert-butyldiméthylsilyl)oxy]-1H-indène ou un analogue à groupe alcool protégé similaire est converti en 1(S)-[1'(S)-hydroxy-éthyl]-7a(S)-méthyl-3a(R)-octahydro-4(R)-[(tert-butyldiméthylsilyl)oxy]-1H-indène ou un analogue à groupe alcool protégé similaire, par exemple par la séquence de réactions avec le méthyllithium, l'oxydation, la réaction de Baeyer-Villiger et la saponification;
b) le 1(S)-[1'(S)-hydroxy-éthyl]-7a(S)-méthyl-3a(R)-octahydro-4(R)-[(tert-butyldiméthylsilyl)-oxy]-1H-indène ou un analogue à groupe alcool protégé similaire est alkylé dans les conditions basiques avec un bloc pour la construction de la chaîne latérale de formule Z-R³, dans laquelle Z est un groupe séparable tel qu'un atome d'halogène, un groupe p-toluènesulfonyloxy ou méthanesulfonyloxy et dans laquelle R³ est R (R est comme défini précédemment ou un analogue) ou éventuellement un radical, qui peut être converti en R pour former un composé de formule VII 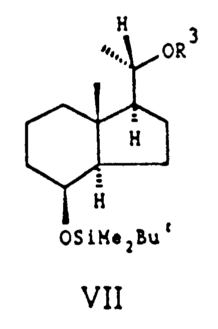 suivi de la conversion du groupe alcool protégé en un groupe céto via la déprotection et l'oxydation, pour donner un composé de formule VIII 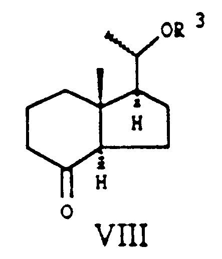
c) Réaction d'un composé de formule VIII avec l'anion dérivé de l'oxyde de [2-[3(S),5(R)-bis-(tert-butyldiméthylsilyloxy)-2-méthylène-cyclohexyl-(Z)-idène]éthyl]diphénylphosphine suivie de la déprotection des groupes alcools, et si nécessaire, conversion de R³ en R ou en un analogue de celui-ci, pour former le composé désiré de formule I de la revendication 1 ou un analogue de celui-ci.

7. Intermédiaire pour la synthèse des composés de formule I et de leurs analogues qui est le
1(S),3(R)-bis(tert-butyldiméthylsilyloxy)-9,10-seco-prégna-5(E), 7(E),10(19)-triène-20-(S)-ol ou le
1(S)-[1'(S)-hydroxyéthyl]-7a(S)-méthyl-3a(R)-octahydro-4(R)-[(tert-butyldiméthylsilyl)oxy]-1H-indène.

8. Une composition pharmaceutique contenant une quantité efficace d'un ou de plusieurs composés selon la revendication 1, ensemble avec des supports et/ou des agents auxiliaires pharmaceutiquement acceptables non toxiques.

9. Une composition pharmaceutique selon la revendication 8, sous forme d'unité de dosage.

10. Une unité de dosage selon la revendication 9, contenant 0,5-500 µg, de préférence 1-250 µg d'un composé de formule I.

11. L'utilisation d'un composé de l'une quelconque des revendications 1 à 4 pour la préparation d'une composition pharmaceutique pour le traitement et la prophylaxie des maladies autoimmunes incluant le diabète sucré, l'hypertension, les maladies inflammatoires telles que la polyarthrite rhumatoïde et l'asthme ainsi que les maladies caractérisées par une différenciation cellulaire anormale et/ou une prolifération cellulaire anormale, et/ou un déséquilibre du système immunitaire.

12. L'utilisation selon la revendication 11 pour le traitement ou la prophylaxie du cancer.

13. L'utilisation selon la revendication 11 pour le traitement du psoriasis.

## Revendications (Revendications pour l'(les) Etat(s) contractant(s) suivant(s): ES)

1. Un procédé de production d'un composé de formule **I** 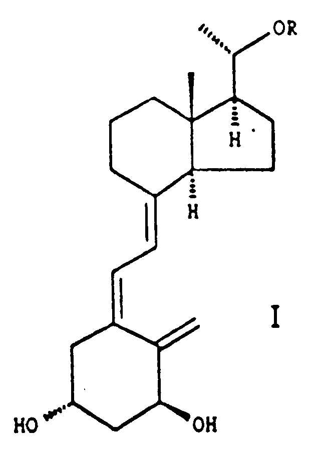 dans laquelle R désigne un groupe alkyle contenant de 7 à 12 atomes de carbone, éventuellement substitué par un groupe hydroxy ; et des dérivés des composés de formule I dans laquelle un ou plusieurs groupes hydroxy ont été transformés en groupes -O-acyle ou O-glycosyle ou ester phosphate, ces groupes masqués étant hydrolysables in vivo, utilisant les étapes suivantes :
a) le 1(S),3(R)-bis-(tert-butyldiméthylsilyloxy)-20(S)-formyl-9,10-seco-prégna-5(E),7(E),10(19)-triène est oxydé par l'oxygène en présence d'un catalyseur basique, pour former la 1(S),3(R)-bis-(tert-butyldiméthylsilyloxy)-9,10-seco-prégna-5(E),7(E),10(19)-triène-20-one;
b) la 1(S),3(R)-bis-(tert-butyldiméthylsilyloxy)-9,10-secoprégna-5(E),7(E), 10(19)-triène-20-one est réduite, par exemple par le borohydrure de sodium, pour former le 1(S),3(R)-bis-(tert-butyldiméthylsilyloxy)-9,10-secoprégna-5(E),7(E), 10(19)-triène-20(S)-ol;
c) le 1(S),3(R)-bis-(tert-butyldiméthylsilyloxy)-9,10-secoprégna-5(E),7(E), 10(19)-triène-20(S)-ol est alkylé dans des conditions basiques avec un bloc pour la construction de la chaîne latérale de formule Z-R³, dans laquelle Z est un groupe séparable tel qu'un atome d'halogène, un groupe p-toluènesulfonyloxy ou méthanesulfonyloxy pour former un composé de formule III 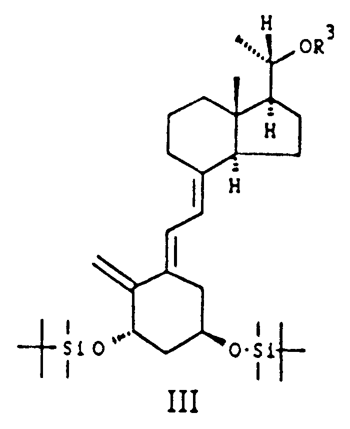 dans laquelle R³ est R (R est comme défini précédemment ou un analogue) ou éventuellement un radical qui peut être converti en R;
d) un composé de formule III précité est soumis à une photoisomérisation avec triplet sansibilisé et, si nécessaire, à une conversion de R³ en R, et une déprotection, pour former le composé désiré de formule I de la revendication 1 ou un analogue de ce dernier,
ou utilisant les étapes suivantes :
a) le 1(S)-[1'(S)-formyléthyl]7a(S)-méthyl-3a(R)-octahydro-4(R)-[(tert-butyldiméthylsilyl)oxy]-1H-indène ou un analogue à groupe alcool protégé similaire est converti en 1(S)-[1'(S)-hydroxy-éthyl]-7a(S)-méthyl-3a(R)-octahydro-4(R)-[(tert-butyldiméthylsilyl)oxy]-1H-indène ou un analogue à groupe alcool protégé similaire, par exemple par la séquence de réactions avec le méthyllithium, l'oxydation, la réaction de Baeyer-Villiger et la saponification;
b) le 1(S)-[1'(S)-hydroxy-éthyl]-7a(S)-méthyl-3a(R)-octahydro-4(R)-[(tert-butyldiméthylsilyl)-oxy]-1H-indène ou un analogue à groupe alcool protégé similaire est alkylé dans les conditions basiques avec un bloc pour la construction de la chaîne latérale de formule Z-R³, dans laquelle Z est un groupe séparable tel qu'un atome d'halogène, un groupe p-toluènesulfonyloxy ou méthanesulfonyloxy et dans laquelle R³ est R (R est comme défini précédemment ou un analogue) ou éventuellement un radical, qui peut être converti en R pour former un composé de formule VII 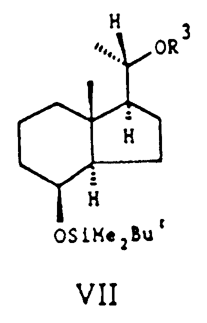 suivi de la conversion du groupe alcool protégé en un groupe céto via la déprotection et l'oxydation, pour donner un composé de formule VIII 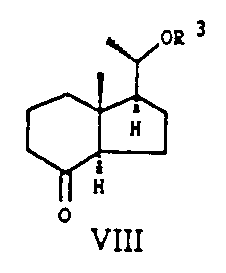
c) Réaction d'un composé de formule VIII avec l'anion dérivé de l'oxyde de [2-[3(S),5(R)-bis-(tert-butyldiméthylsilyloxy)-2-méthylène-cyclohexyl-(Z)-indène]éthyl]diphénylphosphine suivie de la déprotection des groupes alcools, et si nécessaire, conversion de R³ en R ou en un analogue de celui-ci, pour former le composé désiré de formule I de la revendication 1 ou un analogue de celui-ci.

2. Un procédé selon la revendication 1, selon laquelle R est un groupe de formule II 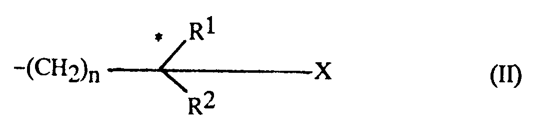 dans laquelle n est un nombre entier de 1 à 7; R¹ et R² qui peuvent être identiques ou différents, désignent un atome d'oxygène, un groupe C₁-C₅-alkyle, C₃-C₇-cycloalkyle ou pris ensemble avec l'atome de carbone (marqué d'une étoile dans la formule II) portant le groupe X, R¹ et R² peuvent former un noyau C₃-C₈ carbocyclique ; et X désigne un groupe hydroxy ou un atome d'hydrogène.

3. Un procédé selon la revendication 1 ou 2, selon lequel un diastéréoisomère d'un composé selon la revendication 1 ou 2, sous forme pure, ou d'un mélange de diastéréoisomères d'un composé selon la revendication 1 ou 2 est préparé.

4. Un procédé selon la revendication 1, selon laquelle on prépare le 1(S),3(R)-dihydroxy-20(S)-(5'-hydroxy-5'-méthyl-1'-hexyloxy)-9,10-seco-prégna-5(Z),7(E),10(19)-triène.

5. Un procédé de production de l'intermédiaire pour la synthèse des composés de formule I et de leurs analogues, qui est le
1(S),3(R)-bis(tert-butyldiméthylsilyloxy)-9,10-seco-prégna-5(E),7(E),10(19)-triène-20-(S)-ol,
selon lequel,
a) le (1(S),3(R)-bis-(tert-butyldiméthylsilyloxy)-20(S)-formyl-9,10-seco-prégna-5(E),7(E),10(19)-triène est oxydé par de l'oxygène en présence d'un catalyseur basique, pour former la 1(S),3(R)-bis-(tert-butyldiméthylsilyloxy)-9,10-seco-prégna-5(E),7(E),10(19)-triène-20-one ; et
b) la 1(S),3(R)-bis-(tert-butyldiméthylsilyloxy)-9,10-seco-prégna-5(E),7(E), 10(19)-triène-20-one est réduite, par exemple par le borohydrure de sodium, pour former le 1(S),3(R)-bis-(tert-butyldiméthylsilyloxy)-9,10-seco-prégna-5(E), 7(E),10(19)-triène-20(S)-ol.

6. Un procédé de production de l'intermédiaire pour la synthèse des composés de formule I ou de leurs analogues, qui est le 1(S)-[1'(S)-hydroxyéthyl]-7a(S)-méthyl-3a(R)-octahydro-4(R)-[(tert-butyldiméthylsilyl)oxy]-1H-indène, selon lequel le 1(S)-[1'(S)-formyléthyl]-7a(S)-méthyl-3a(R)-octahydro-4(R)-[(tert-butyldiméthylsilyl)-oxy]-1H-indène ou un analogue à groupe alcool protégé similaire est converti en composé désiré, par exemple par la séquence de réactions avec le méthyl-lithium, l'oxydation, la réaction de Baeyer-Villiger et la saponification.

7. Un procédé de production d'une composition pharmaceutique qui comprend l'incorporation d'une quantité efficace de un ou de plusieurs composés tels que définis dans la revendication 1, dans des supports et/ou des agents auxiliaires pharmaceutiquement acceptables, non toxiques.

8. Un procédé selon la revendication 7, selon laquelle la composition est préparée sous forme d'unité de dosage.

9. Un procédé selon la revendication 8, selon laquelle l'unité de dosage contient 0,5-500 µg, de préférence 1-250 µg d'un composé de formule I.
